# EUROPÄISCHE PATENTSCHRIFT

(11) **EP 3 202 450 B1**
(45) Veröffentlichungstag und Bekanntmachung des Hinweises auf die Patenterteilung: **12.02.2020**
(21) Anmeldenummer: 17160702.1
(22) Anmeldetag: 03.05.2013
(51) Int. Cl.: A61M 21/00, A61M 21/02, A61B 7/00, A61H 1/00, A61B 5/11, A61G 11/00, A61H 23/02, A61B 5/113

(54) **VORRICHTUNG ZUM ERZEUGEN VON SINNESREIZEN FÜR EIN KIND**
DEVICE FOR GENERATING SENSORY STIMULI FOR A CHILD
DISPOSITIF DE PRODUCTION DE STIMULI SENSORIELS POUR UN ENFANT

(30) Priorität: 03.05.2012 DE 102012103862
(43) Veröffentlichungstag der Anmeldung: 09.08.2017
(62) Teilanmeldung aus: 13166341.1
(73) Patentinhaber: Babybe GmbH, 72631 Aichtal (DE)
(72) Erfinder: Anabalón Alamos, Herr Camilo Andres, 7830238 Santiago (CL); Lang, Herr Raphael Patrick Manfred, 72631 Aichtal (DE)
(74) Vertreter: Wimmer, Stephan

(56) Entgegenhaltungen:
- EP-A2- 1 473 013
- WO-A1-2011/063077
- GB-A- 2 359 994
- US-A1- 2005 171 417
- US-B1- 7 475 441

## Beschreibung

Die vorliegende Erfindung ist auf eine Emulation bzw. Nachbildung von Sinnesreizen, die von einer Bezugsperson ausgehen, für ein von der Bezugsperson räumlich beabstandetes Kind gerichtet. Insbesondere ist die vorliegende Erfindung auf Vorrichtungen, ein System und ein Verfahren zum Erzeugen von Sinnenreizen, die den von einer Bezugsperson ausgehenden Sinnesreizen entsprechen, für ein Kind gerichtet.

Die Geburt stellt für einen Menschen eine drastische Veränderung aller seiner Lebensbedingungen dar. Der unmittelbare physische Kontakt des Neugeborenen zu seiner Mutter, an deren Wärme, Geruch, Stimme, Bewegungen und Herzschlag er bereits gewöhnt ist, ist für ihn deshalb besonders wichtig und wertvoll. Aus medizinischen und anderen Gründen ist dieser unmittelbare physische Kontakt jedoch nicht immer möglich. Insbesondere nach einer Frühgeburt muss das Neugeborene oft viele Wochen in einem Inkubator verbringen.

Ein Inkubator bzw. Brutkasten stellt für ein Neugeborenes einen hygienischen Raum mit einem vorteilhaften Mikroklima dar, insbesondere mit optimaler Temperatur und Luftfeuchtigkeit. Die dem Neugeborenen aus der Zeit vor seiner Geburt vertrauten Sinnesreize fehlen jedoch, insbesondere die Bewegungen, die Stimme, die Herztöne und der Geruch der Mutter. Es existieren zahlenreiche Ansätze, um einen Säugling oder einen anderen Menschen entsprechenden Sinnesreizen auszusetzen.

In US 3,672,354 ist eine Vorrichtung mit einem aufblasbaren Polster beschrieben. Ein Luftverdichter erzeugt Druckpulse mit der Frequenz des menschlichen Herzschlags.

In US 3,809,065 ist ein System beschrieben, um ein Neugeborenes sich langsam ändernden Umweltbedingungen auszusetzen. Die Temperatur, taktil Wahrnehmbares, Geräusche, Bewegung und Licht werden langsam von im Mutterleib herrschenden bis zu den außerhalb des Mutterleib herrschenden Bedingungen verändert.

In US 2011/0144416 A1 ist eine Vorrichtung beschrieben zum Erzeugen rhythmischer Bewegungen ähnlichen denen, die ein Kind am Brustkorb seiner Mutter erlebt.

Herkömmliche Vorrichtungen, wie die beschriebenen, vermitteln einem Säugling jedoch lediglich synthetische Sinnesreize, die den unmittelbaren physischen Kontakt des Säuglings mit seiner Mutter oder seinem Vater nicht ersetzen können. Herkömmliche Vorrichtungen sind oft auch mechanisch aufwändig ausgestaltet, ohne aus Sicht des Säuglings einen auch nur näherungsweise vergleichbaren Ersatz für den unmittelbaren Kontakt mit Mutter oder Vater zu schaffen. Herkömmliche Vorrichtungen sind oft nur eingeschränkt für einen Inkubator geeignet, weil sie für Röntgenstrahlung nicht oder nicht in ausreichendem Maße transparent sind.

Herkömmliche Vorrichtungen sind oft nicht für den Einsatz im Inkubator geeignet da sie durch eine geringe Wärmeleitfähigkeit der verwendeten Materialien und durch ihre Bauart das Mikroklima im Inkubator und vor allem das elektrische Wärmeregelsystem des Inkubators negative beeinflussen oder stören. Herkömmliche Vorrichtungen sind oft auch mechanisch aufwändig ausgestaltet und erzeugen daher mechanische Störgeräusche oder Vibrationen, die eine schädliche akustische Umgebung, insbesondere eine zu hohe Lautstärke, für den Säugling im Inkubator erzeugen.

Herkömmliche Vorrichtungen sind oft mit großen Öffnungen oder offenen Höhlräumen oder Mulden oder mit Verbindungselementen wie Klettverschlüssen oder Reißverschlüssen versehen. An oder in diesen können sich aufgrund des Mikroklimas des Inkubators, insbesondere der hohen Luftfeuchtigkeit und Temperatur, Kondenswasser bilden und Keime ansiedeln. Herkömmliche Vorrichtungen können dadurch die Sterilität des Innenraums des Inkubators aufheben und eine hohe Gefahr für frühgeborene Kinder darstellen.

Herkömmliche Vorrichtungen weisen oft elektrische Bauteile wie Lautsprecher, Motoren und andere Aktoren auf, die insbesondere direkt unter der Liegefläche des Säuglings angeordnet sind. Diese elektrischen Bauteile stellen bei einem möglichen elektrischen Kurzschluss mit der daraus resultierenden Wärmeentwicklung und Brandgefahr ein konzeptionelles Risiko dar und sind deshalb eigentlich nicht oder nur eingeschränkt für den Betrieb im Inneren eine Inkubators geeignet.

Herkömmliche Vorrichtungen sind oft auch mechanisch aufwändig ausgestaltet und weisen eine Vielzahl von verwendeten Materialen auf. Insbesondere herkömmliche Vorrichtungen mit integrierten Pneumatik- oder Hydraulik-Zylindern oder Motoren können Schmierstoffe und andere bedenkliche Stoffe enthalten. Schmierstoffe und andere Materialien können insbesondere in dem feuchtwarmen Mirkoklima des Inkubators ausgasen und bei einem Säugling beispielsweise Allergien auslösen oder sich im Blut anreichern und gesundheitsschädliche Wirkung entwickeln.

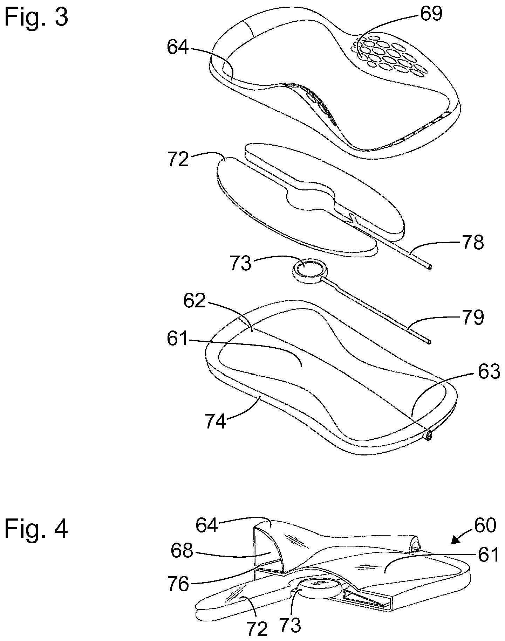

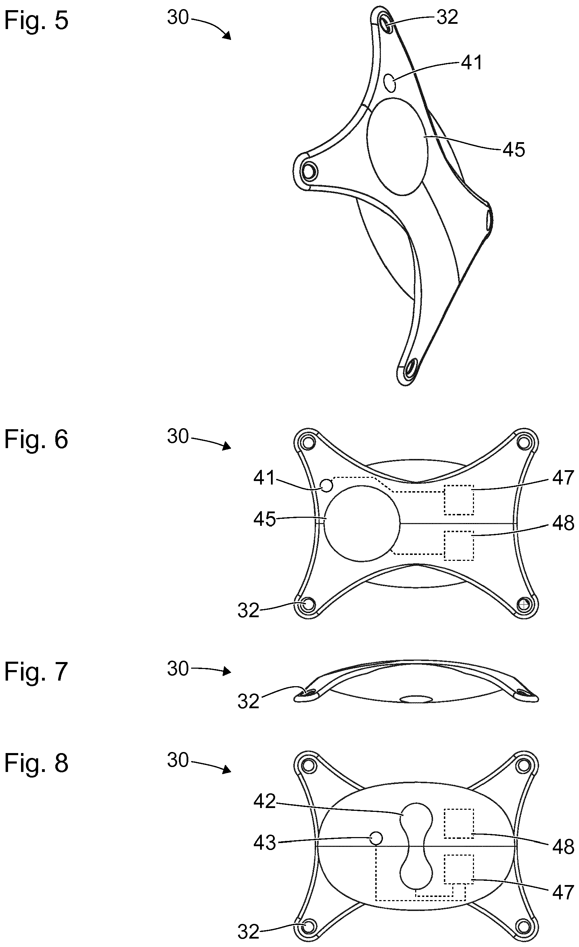

In WO 2022/063077 A1 ist eine Vorrichtung 10B, 10C zur Simulation von Schall und Bewegungen im Uterus beschrieben (Absatz [0056], Figuren 3A, 5). Die Vorrichtung umfasst eine abgedichtete äußere Umhüllung 14 auf einer flachen Oberfläche eines Stützelements 60 (Absatz [0062], Figur 3A). Eine Bewegungsaktivierungseinheit 42 ist innerhalb der abgedichteten äußeren Umhüllung 14 angeordnet (implizit in Paragraph [0058], Figur 3A). Die Bewegungsaktivierungseinheit 42 umfasst Gang- oder Körperbewegungsbälge oder -kissen 44 und eine Atmungsblase oder ein Atmungskissen 46 (Absatz [0058], Figur 3A).

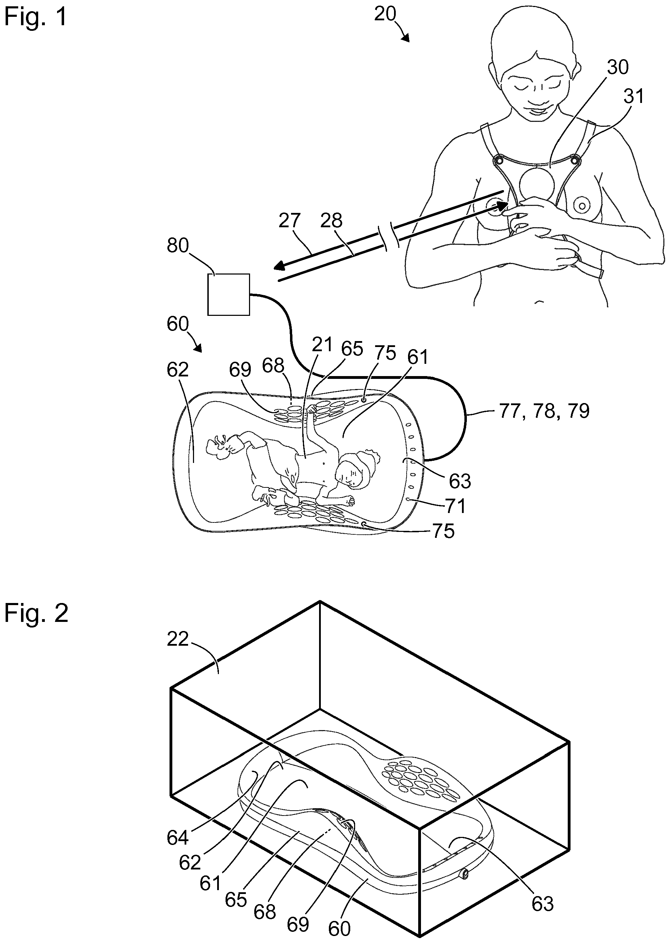

In GB 2 359 994 A ist eine Vorrichtung zur Simulation des Herzschlags einer Mutter für ein Kind beschrieben, das in einem Bett liegt (Seite 1, Zeilen 12 und 13). Die Vorrichtung umfasst eine hohle flexible Blase 14, die zwischen einer Matratze 10 und einer Decke 12 angeordnet ist (Seite 4, Zeilen 9 bis 11; Figuren 1, 2).

In EP 1 473 013 A2 ist ein vibrierendes Patientenlagerungssystem mit einer Blase 14 und einem Vibrationskissen 12 in einer Polstereinrichtung 10 beschrieben (Absätze [0007], [0017]). Das Vibrationskissen 12 ist unter der unteren Oberfläche der Blase 14 oder innerhalb der Blase 14 und unter der oberen Oberfläche der Blase 14 angeordnet (Absätze [0007], [0017]).

In US 7,475,441 B1 ist eine Babytröstungsvorrichtung 10 beschrieben, auf der ein Baby liegen kann (Spalte 3, Zeilen 21 und 22). Die Vorrichtung 10 umfasst eine Plattform 18 und eine elastische Polsterung 16 auf der Oberseite der Plattform 18 (Spalte 3, Zeilen 28 bis 36). Ein Scharnier 22 verbindet ein erstes Ende der Plattform 18 mit einer Basis 20 (Spalte 3, Zeilen 47 und 48). Ein Bewegungszylinder 30 bewegt das zweite Ende der Plattform 18 relativ zu der Basis 20 (Spalte 3, Zeilen 48 bis 51).

Eine Aufgabe der vorliegenden Erfindung besteht darin, eine verbesserte Vorrichtung zum Erzeugen von Sinnesreizen, die den von einer Bezugsperson ausgehenden Sinnesreizen entsprechen, zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, die von einem Elternteil (Mutter, Vater, Stief- oder Pflegemutter, Stief- oder Pflegevater) oder einer anderen Bezugsperson erzeugten Sinnesreize mittels Sensoren zu erfassen, in Signale bzw. einen Datenstrom zu wandeln, diesen zum Kind zu übertragen und gesteuert durch die übertragenen Signale bzw. den übertragenen Datenstrom das Kind entsprechenden Sinnesreizen auszusetzen. Die Sinnesreize, denen das Kind ausgesetzt ist, sind also nicht nur technisch-synthetischer Natur, sind nicht nur aufgezeichnet und reproduziert und sind nicht standardisiert. Vielmehr werden die tatsächlich von der Mutter (oder dem Vater oder einer anderen Bezugsperson ausgehenden) Sinnesreize erfasst, als Datenstrom übertragen und beim Kind nachgebildet. Somit erfährt das Kind die spezifischen und hinsichtlich ihrer Eigenschaften individuellen und charakteristischen Sinnesreize, die von seiner lebendigen Mutter ausgehen. Damit bleiben die individuellen und dem Kind vertrauten Charakteristika der Sinnesreize bis hin zu ihrer Variabilität und den Eigenschaften ihrer Zeitabhängigkeit erhalten. Damit wird eine völlig neue Qualität der Simulation des unmittelbaren physischen Kontakts zwischen Kind und Bezugsperson, insbesondere zwischen Säugling und Mutter geschaffen.

Eine Vorrichtung zum Erzeugen von Sinnesreizen, die den von einer Bezugsperson ausgehenden Sinnesreizen entsprechen, für ein Kind umfasst eine Empfangseinrichtung zum Empfangen eines Datenstroms von einer Vorrichtung zum Erfassen von von einer Bezugsperson ausgehenden Sinnesreizen und eine Wandlereinrichtung, die mit der Empfangseinrichtung gekoppelt und durch den Datenstrom steuerbar ist, zum Erzeugen von zumindest entweder Körperschall, der durch den Datenstrom beschriebenen Herztönen der Bezugsperson entspricht, oder Bewegungen, die durch den Datenstrom beschriebenen Bewegungen der Bezugsperson entsprechen, oder Luftschall, der von der Bezugsperson erzeugtem und durch den Datenstrom beschriebenem Luftschall entspricht.

Die Bezugsperson ist insbesondere die leibliche Mutter, die Adoptiv-, Stief- oder Pflegemutter, der leibliche Vater, der Adoptiv-, Stief- oder Pflegevater. Die Bezugsperson kann aber auch ein (insbesondere deutlich älteres) Geschwister oder eine andere Bezugsperson sein. Das Kind ist insbesondere ein Säugling oder ein Kleinkind oder ein Kind beliebigen Alters mit ausgeprägter emotionaler Beziehung zu einer Bezugsperson, beispielsweise ein Kind, dessen geistige und/oder emotionale Entwicklung aufgrund einer Erkrankung oder einer Behinderung retardiert ist.

Die Empfangseinrichtung ist zum Empfangen eines Datenstroms ausgebildet, der von einer Vorrichtung zum Erfassen und Übertragen von von einer Bezugsperson ausgehenden Sinnesreizen bereitgestellt wird. Die Empfangseinrichtung ist somit insbesondere zum Empfangen eines kontinuierlich in Echtzeit bzw. mit geringer Zeitverzögerung oder mit einer konstanten Zeitverzögerung erzeugten und übertragenen Datenstroms ausgebildet. Dadurch unterscheidet sich die Vorrichtung insbesondere wesentlich von einer Vorrichtung, bei der lediglich aufgezeichnete und gespeicherte Sinnesreize reproduziert werden.

Die Vorrichtung kann eine oder mehrere Wandlereinrichtungen zum Erzeugen von Körperschall und/oder Bewegungen und/oder Luftschall aufweisen. Sofern die Wandlereinrichtung zum Erzeugen von Bewegungen ausgebildet ist, ist sie insbesondere zum Erzeugen von Bewegungen, die durch den Datenstrom beschriebenen Brustkorbbewegungen der Bezugsperson entsprechen, ausgebildet.

Mit Körperschall ist Schall gemeint, der sich überwiegend innerhalb eines Festkörpers oder einer Flüssigkeit, insbesondere innerhalb des Körpers der Bezugsperson oder innerhalb eines Gegenstands, auf dem das Kind liegt, ausbreitet. Mit Luftschall ist Schall gemeint, der sich überwiegend in Luft ausbreitet. Ohne besondere Maßnahmen, insbesondere ohne eine zumindest weitgehende Impedanzanpassung findet eine Übertragung von Leistung zwischen Körperschall und Luftschall oft nur in geringem Maße statt. Körperschall und Luftschall werden in der Regel unterschiedlich wahrgenommen. Luftschall wird unabhängig von einem unmittelbaren Körperkontakt über die Gehörgänge mittels der Ohren wahrgenommen, Körperschall wird bei unmittelbarem oder mittelbarem Körperkontakt nicht nur mittels der Ohren, sondern unter Umständen auch taktil wahrgenommen.

Luftschall wird von einer Bezugsperson insbesondere mittels seiner Stimme, durch Reden, Singen, Summen, Schnalzen, Pfeifen etc. erzeugt. Körperschall wird von einer Bezugsperson insbesondere in Form von Herztönen und vom Verdauungstrakt ausgehenden Geräuschen erzeugt. Auch die Stimme der Bezugsperson wird teilweise in Form von Körperschall auf ein Kind übertragen, der unmittelbaren Körperkontakt zur Bezugsperson hat

Entsprechend den unterschiedlichen Orten und Mechanismen der Entstehung und entsprechend den resultierenden unterschiedlichen Frequenzspektren, Amplituden, Intensitäten sind insbesondere zwei verschiedene und unterschiedliche Wandlereinrichtungen zum Erzeugen von Körperschall einerseits und Luftschall andererseits vorgesehen.

Bei einer Vorrichtung, wie sie hier beschrieben ist, umfasst die Wandlereinrichtung insbesondere zumindest entweder einen Schallgenerator zum Erzeugen von Körper- oder Luftschall oder einen Verdichter oder eine Pumpe für ein Fluid oder einen Balg oder einen Motor zum Erzeugen von Bewegungen.

Als Schallgenerator zum Erzeugen von Luftschall ist insbesondere ein Lautsprecher vorgesehen. Als Schallgenerator zum Erzeugen von Körperschall oder als Wandlereinrichtung zum Erzeugen Vibrationen ist insbesondere eine Pendelpumpe oder ein mit einem elektromechanischen Antrieb versehener Steuerbalg, der mit einem Balg an einer Liegefläche für das Kind verbunden ist, vorgesehen. Als Verdichter ist insbesondere ein Ventilator oder ein Gebläse vorgesehen. Die Wandlereinrichtung kann eine Einheit bilden oder mehrere, insbesondere fluidisch und/oder mittels elektrischer oder optischer Signalleitungen miteinander gekoppelte Einheiten für eine Anordnung an mehreren voneinander beabstandeten Orten aufweisen.

Als Motor zum Antrieb eines Steuerbalgs, der beispielsweise mit einem Balg zum Bewegen einer Liegefläche fluidisch verbunden ist, oder zum unmittelbaren Antrieb einer Liegefläche kann ein Elektromotor, insbesondere ein Schrittmotor verwendet werden. Ein Elektromotor kann als Linearmotor oder als rotierende und insbesondere mit einem Getriebe kombinierte Maschine ausgebildet sein.

Eine Vorrichtung, wie sie hier beschrieben ist, umfasst insbesondere ferner eine Liegefläche für ein Kind.

Insbesondere umfasst die Vorrichtung eine Matratze oder eine Matte oder eine andere Unterlage, deren obere Oberfläche die Liegefläche für das Kind bildet. Alle weiteren Bestandteile der Vorrichtung können in diese Matratze oder Matte oder Unterlage integriert oder für eine separate Anordnung ausgebildet sein. Insbesondere sind die Matratze oder Matte oder Unterlage für eine Anordnung innerhalb eines Inkubators und weitere Bestandteile der Vorrichtung für eine Anordnung außerhalb des Inkubators vorgesehen.

Bei einer Vorrichtung mit einer Liegefläche, wie sie hier beschrieben ist, umfasst die Wandlereinrichtung insbesondere einen Balg unter der Liegefläche und eine Steuereinrichtung, wobei die Steuereinrichtung mit dem Balg fluidisch gekoppelt ist und eine Pumpe oder einen Verdichter oder einen Steuerbalg umfasst.

Insbesondere sind zwei Balge symmetrisch oder im Wesentlichen symmetrisch nebeneinander unter der Liegefläche angeordnet, ähnlich den Lungenflügeln des Menschen. Ein oder mehrere mit Luft oder einem anderen Gas gefüllte Balge unter der Liegefläche können eine gleichmäßige, großflächige, geräusch- und erschütterungsarme vertikale Bewegung der Liegefläche ermöglichen.

Alternativ oder zusätzlich kann ein Balg oder können mehrere Balge vorgesehen und angeordnet sein, um Körperschall oder Vibrationen - insbesondere entsprechend dem Herzschlag der Bezugsperson - in oder unter der Liegefläche zu erzeugen. Zur Erzeugung von Körperschall ist ein Balg insbesondere mit einer Flüssigkeit gefüllt.

Bei einer Vorrichtung mit einer Steuereinrichtung, wie sie hier beschrieben ist, ist die Steuereinrichtung insbesondere von der Liegefläche räumlich beabstandet und mit der Liegefläche mittels einer Fluidleitung verbunden.

Die Steuereinrichtung umfasst insbesondere einen oder mehrere Steuerbalge, Pumpen, Verdichter oder andere Einrichtungen zum Übertragen von Leistung auf ein Fluid und ist für eine Anordnung außerhalb eines Inkubators vorgesehen und ausgebildet. Eine Fluidleitung zur fluidischen Kopplung der Steuereinrichtung mit dem Balg oder den Balgen umfasst insbesondere ein oder mehrere Rohre und/oder einen oder mehrere Schläuche.

Eine von der Liegefläche beabstandete Steuereinrichtung kann eine Verwendung der Vorrichtung mit einem herkömmlichen Inkubator ermöglichen.

Bei einer Vorrichtung, wie sie hier beschrieben ist, sind insbesondere die Liegefläche und der Balg für eine Anordnung innerhalb eines Inkubators und die Steuereinrichtung und die Empfangseinrichtung für eine Anordnung außerhalb eines Inkubators ausgebildet.

Für eine Anordnung innerhalb eines Inkubators sind der Balg und insbesondere die Liegefläche insbesondere sterilisierbar oder als Einweg-Produkte vorgesehen und ausgebildet, während die Steuereinrichtung und die Empfangseinrichtung aufgrund ihrer Anordnung außerhalb eines Inkubators nicht sterilisierbar sein müssen.

Eine Vorrichtung, wie sie hier beschrieben ist, umfasst insbesondere ferner einen Hohlraum zum Aufnehmen eines als Geruchsquelle dienenden Gegenstands.

Der Hohlraum ist insbesondere am Rand der Liegefläche vorgesehen. Der Hohlraum ist insbesondere zur Aufnahme eines Handtuchs oder eines anderen Gegenstands, der den Geruch der Bezugsperson angenommen hat und langsam abgibt, vorgesehen. Der Hohlraum weist insbesondere eine oder mehrere Öffnungen auf, durch die hindurch ein Gasaustausch stattfindet. Der Gasaustausch kann durch einen Ventilator oder ein Gebläse unterstützt sein. Durch eine Anordnung einer Geruchsquelle in dem Hohlraum können auch hinsichtlich der olfaktorischen Wahrnehmung vertraute Umgebungsbedingungen für das Kind geschaffen werden.

Eine Vorrichtung, wie sie hier beschrieben ist, umfasst ferner insbesondere einen Sensor oder einen Signaleingang zum Erfassen zumindest entweder von Schall, der von dem Kind erzeugt wird, oder von Bewegungen des Kinds oder eines Zustands des Kinds und zum Erzeugen eines weiteren Datenstroms, der den Schall bzw. die Bewegungen bzw. den Zustand beschreibt, und eine Übertragungseinrichtung zum Übertragen des weiteren Datenstroms zu einer Empfangseinrichtung.

Als Sensor zum Erfassen von Schall ist insbesondere ein Mikrofon zum Erfassen von Luft- oder Körperschall vorgesehen. Als Signaleingang ist insbesondere ein analoger oder digitaler Signaleingang zum Empfangen von Daten von einem oder mehreren medizinischen Instrumenten vorgesehen. Der Zustand des Kinds umfasst insbesondere seinen Gesundheitszustand, beispielsweise seine Körpertemperatur, seinen Puls, seinen Blutdruck, die Sauerstoffsättigung seines Bluts, seine Atemfrequenz oder daraus abgeleitete oder berechnete Parameter. Der Zustand des Kinds kann ferner Parameter umfassen, die sein Wohlbefinden, seinen Schlaf- oder Wachzustand beschreiben. Die Übertragungseinrichtung ist insbesondere zum Übertragen des weiteren Datenstroms zu einer Empfangseinrichtung bei einer Bezugsperson vorgesehen.

Die zuletzt beschriebenen Merkmale können eine bidirektionale Wechselwirkung zwischen Bezugsperson und Kind ermöglichen. Insbesondere können Reaktionen des Kinds auf von der Bezugsperson ausgehende Sinnesreize an die Bezugsperson zurück übermittelt werden. Ferner können Reaktionen der Bezugsperson auf vom Kind ausgehende Sinnesreize, auf den Gesundheitszustand des Kinds oder auf Äußerungen des Kinds von der Bezugsperson zum Kind übermittelt werden. Damit kann eine Interaktion zwischen Bezugsperson und Kind ermöglicht werden, die der bei einem unmittelbaren physischen Kontakt ähnlich ist. Beispielsweise kann die Bezugsperson auf das Befinden des Kinds reagieren und seinerseits das Kind durch durch Bewegungen, Ansprache und selbst durch rein vegetativ gesteuerte Vorgänge, beispielsweise den Herzschlag der Bezugsperson, beeinflussen.

Bei einer Vorrichtung, wie sie hier beschrieben ist, entspricht die Anordnung der Wandlereinrichtung oder der Wandlereinrichtungen relativ zu einer vorgesehenen Anordnung und Ausrichtung des Kinds der Anordnung der Quellen der Sinnesreize an der Bezugsperson.

Insbesondere sind beispielsweise eine Wandlereinrichtung zum Erzeugen von Luftschall bzw. ein Lautsprecher an einem Kopfende einer Liegefläche der Vorrichtung, eine Wandlereinrichtung zum Erzeugen von Körperschall in der Mitte der Liegefläche angeordnet und eine Wandlereinrichtung zum Erzeugen von vertikalen Bewegungen der Liegefläche für eine großflächige Bewegung der Liegefläche ausgebildet. Somit kann ein Kind die Sinnesreize jeweils im Wesentlichen aus der Richtung erhalten, aus der es sie erhalten würde, wenn es beispielsweise auf dem Brustkorb der Bezugsperson läge.

Bei einer Vorrichtung, wie sie hier beschrieben ist, sind insbesondere eine erste Wandlereinrichtung zum durch den Datenstrom gesteuerten Heben und Senken eines Bereichs der Liegefläche, der mindestens ein Drittel der Liegefläche umfasst, ausgebildet, eine zweite Wandlereinrichtung zum durch den Datenstrom gesteuerten Erzeugen von Körperschall oder Vibrationen in der Liegefläche ausgebildet und in einem mittleren Bereich der Liegefläche angeordnet und eine dritte Wandlereinrichtung zum durch den Datenstrom gesteuerten Erzeugen von Schall nahe einem für einen Kopf eines Kind vorgesehenen Bereich angeordnet.

Die erste Wandlereinrichtung ist insbesondere zum durch den Datenstrom gesteuerten Heben und Senken eines Bereichs der Liegefläche ausgebildet, der mindestens die Hälfte oder mindestens zwei Drittel oder im Wesentlichen die gesamte Liegefläche umfasst.

Eine Vorrichtung, wie sie hier beschrieben ist, umfasst insbesondere eine Lichtquelle zum Erzeugen von Licht und zum Bestrahlen eines Kinds mit dem Licht.

Das Licht kann für therapeutische Zwecke vorgesehen sein und dafür geeignete Eigenschaften (insb. Wellenlänge, Intensität) aufweisen. Die Lichtquelle ist gegebenenfalls insbesondere in die beschriebene Liegefläche integriert, um ein auf der Liegefläche liegendes Kind zu bestrahlen. Die Lichtquelle kann ausgebildet sein, um Licht mit im Wesentlichen homogener Leuchtdichte oder Licht mit im Wesentlichen homogener Intensität aus der gesamten Liegefläche oder aus einem vorbestimmten Bereich der Liegefläche austreten zu lassen. Alternativ kann die Lichtquelle ausgebildet sein, um lediglich in einem Bereich der Liegefläche Licht zu erzeugen oder austreten zu lassen, in dem ein Kind auf der Liegefläche liegt. Dazu kann die Vorrichtung insbesondere Eigenschaften und Merkmale aufweisen, wie sie in US 2007/0088410 A1 beschrieben sind.

Insbesondere eine Kombination von einem oder mehreren Balgen, wie sie hier beschrieben sind, unter der Liegefläche mit einer Lichtquelle ist vorteilhaft, da der oder die Balgen transparent für das Licht der Lichtquelle ausgebildete sein können. Die Lichtquelle kann somit unter dem oder den Balgen angeordnet sein. Deshalb muss die Lichtquelle an der Bewegung der Liegefläche nicht teilnehmen und kann starr ausgebildet und angeordnet sein.

Eine Vorrichtung zum Erfassen und Übertragen von von einer Bezugsperson ausgehenden Sinnesreizen umfasst einen Sensor zumindest entweder zum Erfassen von Herztönen der Bezugsperson oder zum Erfassen von Brustkorbbewegungen der Bezugsperson oder zum Erfassen von durch die Bezugsperson erzeugten Luftschall, und zum Erzeugen eines Datenstroms, der die Herztöne bzw. die Brustkorbbewegungen bzw. den Luftschall beschreibt, und eine Übertragungseinrichtung zum Übertragen des Datenstroms zu einer Vorrichtung zum Erzeugen von Sinnesreizen, die den von der Bezugsperson ausgehenden Sinnesreizen entsprechen, für ein Kind.

Mit dem Erfassen von Herztönen ist insbesondere nicht nur das Erfassen des Pulses der Bezugsperson gemeint. Vielmehr sind der Köperschall und/oder die Vibrationen, die durch die Tätigkeit des Herzens der Bezugsperson im Brustkorb der Bezugsperson erzeugt und von einem am Brustkorb der Bezugsperson liegenden Kind wahrgenommen werden kann, gemeint. Die Übertragungseinrichtung ist insbesondere zum Übertragen des Datenstroms zu einer Vorrichtung zum Erzeugen von Sinnesreizen, wie sie oben beschrieben ist, ausgebildet.

Eine Vorrichtung zum Erfassen und Übertragen von von einer Bezugsperson ausgehenden Sinnesreizen umfasst insbesondere ferner eine Empfangseinrichtung zum Empfangen eines weiteren Datenstroms und eine Wandlereinrichtung, die mit der Empfangseinrichtung gekoppelt und durch den weiteren Datenstrom steuerbar ist, wobei die Wandlereinrichtung ausgebildet ist, um zumindest entweder Schall, der von dem Kind erzeugten und durch den weiteren Datenstrom beschriebenem Schall entspricht, zu erzeugen oder Bewegungen, die durch den weiteren Datenstrom beschriebenen Bewegungen des Kinds entsprechen, zu erzeugen oder einen durch den Datenstrom beschriebenen Zustand des Kinds anzuzeigen.

Der durch den Datenstrom beschriebene Zustand des Kinds kann grafisch, insbesondere mittels Symbolen, alphanumerisch, optisch, akustisch, haptisch und/oder auf andere Weise angezeigt werden. Dies kann eine Rückmeldung vom Kind zu der Bezugsperson und damit eine bidirektionale Interaktion zwischen Bezugsperson und Kind ermöglichen.

Eine Vorrichtung zum Erfassen und Übertragen von von einer Bezugsperson ausgehenden Sinnesreizen ist insbesondere ausgebildet, um von einer Bezugsperson am Brustkorb getragen zu werden.

Die Vorrichtung ist insbesondere hinsichtlich ihrer räumlich-geometrischen Gestalt, ihrer Masse sowie hinsichtlich ihrer Oberflächeneigenschaften ausgebildet, um ähnlich wie ein Säugling am Brustkorb getragen zu werden und dabei in einer an einen Säugling erinnernden Weise wahrgenommen zu werden.

Eine Vorrichtung zum Erfassen und Übertragen von von einer Bezugsperson ausgehenden Sinnesreizen, wie sie hier beschrieben ist, weist insbesondere einen Tragegurt oder eine Befestigungseinrichtung für einen Tragegurt auf.

Ein System umfasst insbesondere eine Vorrichtung zum Erzeugen von Sinnesreizen, wie sie hier beschrieben ist, und eine Vorrichtung zum Erfassen und Übertragen von von einer Bezugsperson ausgehenden Sinnesreizen, wie sie hier beschrieben ist.

Die hier beschriebenen Vorrichtungen und das hier beschriebene System sind insbesondere ausgebildet, um den Datenstrom und ggf. auch den weiteren Datenstrom analog und/oder digital und in Form von elektrischen, optischen, elektromagnetischen, akustischen oder anderen Signalen auszutauschen. Ein Übertragungskanal zwischen beiden Vorrichtungen kann eine elektrisch und/oder optisch Punkt-Zug-Punkt-Verbindung, einen elektrischen oder optischen Bus, das Internet, ein Telefonnetz oder ein anderes privates oder öffentliches Netzwerk umfassen und Technologien wie WLAN, WiFi, GSM, UMTS und/oder andere Technologien umfassen. Insbesondere bei einem bidirektionalen Datenaustausch, bei dem sowohl ein Datenstrom von der Bezugsperson zum Kind als auch ein weiterer Datenstrom vom Kind zu der Bezugsperson übertragen wird, findet die Übertragung insbesondere in Echtzeit bzw. mit einer möglichst kurzen Verzögerung, die wenige Sekunden oder weniger beträgt, statt.

Bei einem Verfahren zum Erzeugen von Sinnesreizen, die den von einer Bezugsperson ausgehenden Sinnesreizen entsprechen, für ein von der Bezugsperson räumlich beabstandeten Kind werden zumindest entweder Herztöne der Bezugsperson oder Bewegungen der Bezugsperson oder durch die Bezugsperson erzeugter Luftschall erfasst, ein Datenstrom, der die Herztöne bzw. die Bewegungen bzw. den Luftschall beschreibt, erzeugt, der Datenstrom von der Bezugsperson zum Kind übertragen und gesteuert durch den Datenstrom den Herztönen entsprechender Körperschall oder den Bewegungen entsprechende Bewegungen oder dem Luftschall entsprechender Luftschall erzeugt.

Der Datenstrom wird insbesondere von einer Übertragungseinrichtung bzw. Sendeeinrichtung an einer Vorrichtung, die die Bezugsperson bei sich trägt, zu einer Empfangseinrichtung einer Vorrichtung beim Kind übertragen. Die erfassten Bewegungen sind insbesondere Bewegungen des Brustkorbs der Bezugsperson.

Bei einem Verfahren, wie es hier beschrieben ist, werden insbesondere ferner zumindest entweder Schall, der von dem Kind erzeugt wird, oder Bewegungen des Kinds oder ein Zustand des Kinds erfasst, ein weiterer Datenstrom, der den Schall oder die Bewegungen oder den Zustand beschreibt, erzeugt, der weitere Datenstrom vom Kind zu der Bezugsperson übertragen und bei der Bezugsperson durch den weitere Datenstrom Schall oder Bewegungen oder ein von der Bezugsperson wahrnehmbares Signal erzeugt.

Der Datenstrom wird insbesondere von einer Übertragungseinrichtung einer Vorrichtung beim Kind zu einer Empfangseinrichtung einer Vorrichtung, die bei der Bezugsperson angeordnet ist, übertragen. Das Wandeln des Datenstroms in ein durch die Bezugsperson wahrnehmbares Signal umfasst insbesondere das Erzeugen einer grafischen, alphanumerischen, akustischen, optischen oder anderen Anzeige des Zustands des Kinds.

Ein Verfahren, wie es hier beschrieben ist, kann insbesondere mit Vorrichtungen, wie sie hier beschrieben sind, ausgeführt werden.

Eine Vorrichtung zum Erzeugen von Sinnesreizen für ein Kind umfasst einen Körper aus einem elastischen Material, eine Liegefläche für ein Kind auf dem Körper und einen Balg zum Erzeugen einer vertikalen Bewegung der Liegefläche, wobei der Balg in das elastische Material des Körpers eingebettet ist.

Der Körper mit der Liegefläche und dem Balg bildet insbesondere eine Matratze zur Anordnung in einem Inkubator. Der Balg oder mehrere Balge sind insbesondere zum Erzeugen einer vertikalen Bewegung, die einer durch die Atmung bedingten Bewegung der Vorderseite des Oberkörpers einer Mutter oder eines anderen erwachsenen Menschen entspricht, und/oder zum Erzeugen von Körperschall, der dem Herzschlag einer Mutter oder eines anderen erwachsenen Menschen entspricht, in dem Körper vorgesehen und ausgebildet. Der Körperschall äußert sich insbesondere in vertikalen Bewegungen der Liegefläche, die sich von einer Atembewegungen entsprechenden Bewegungen in der Frequenz, der Amplitude, der räumlichen Verteilung und der Zeitabhängigkeit unterscheiden können.

Das elastische Material des Körpers weist insbesondere eine Massendichte auf, die derjenigen des menschlichen Körpers bzw. von Muskel- oder Fettgewebe des menschlichen Körpers ähnlich ist. Das elastische Material des Körpers weist insbesondere eine hohe Wärmeleitfähigkeit auf, um eine gleichmäßige Temperierung des Körpers, beispielsweise in einem Inkubator, zu ermöglichen. Das elastische Material des Körpers umfasst insbesondere Polyurethan-Gummi, Polyurethan-Gel, Latex oder ein anderes elastisches Material.

Bei einer Vorrichtung mit einem Körper aus einem elastischen Material, wie sie hier beschrieben ist, weist der Körper insbesondere von dem Balg oder von mehreren Balgen abgesehen keine weiteren Hohlräume auf.

Indem der Körper keine weiteren Hohlräume aufweist, sind eine Verschmutzung von Hohlräumen und eine Besiedelung von Hohlräumen mit Keimen ausgeschlossen. Die Vorrichtung kann deshalb besonders einfach zu reinigen und zu sterilisieren sein.

Eine Vorrichtung mit einem Körper aus einem elastischen Material, wie sie hier beschrieben ist, ist erfindungsgemäß durch ein Gießverfahren hergestellt, bei dem das elastische Material des Körpers mit der elastischen Folie und mit dem Balg vollflächig oder im Wesentlichen vollflächig stoffschlüssig verbunden wird.

Dabei werden insbesondere keine Hohlräume erzeugt. Das elastische Material, die elastische Folie und der Balg bilden also eine monolithische und auch bei über einen langen Zeitraum wiederholter elastischer Verformung untrennbare Einheit, die von der inneren Oberfläche des Balgs oder der Balgen abgesehen nur äußere Oberflächen aufweist, die leicht gereinigt und sterilisiert werden können.

Eine Vorrichtung mit einem Körper aus einem elastischen Material, wie sie hier beschrieben ist, umfasst insbesondere ferner eine elastische Folie an der Oberfläche des Körpers, die zumindest die Liegefläche bildet.

Die elastische Folie weist insbesondere Polyurethan oder Polycabonat oder ein ähnliches elastisches Material auf. Die elastische Folie ist so elastisch, dass sie durch den Balg oder die Balgen ohne Weiteres zusammen mit dem Körper elastisch verformt werden kann.

Die elastische Folie weist insbesondere eine geschlossene bzw. porenfreie oder porenarme und leicht abwischbare Oberfläche auf. Die elastische Folie ist insbesondere gasdicht. Die elastische Folie kann bakteriostate, bakterizide, virustate, viruzide und/oder andere keimtötende Eigenschaften aufweisen. Die elastische Folie kann die gesamte Oberfläche des Körpers bedecken. Die elastische Folie kann die Reinigung und/oder Sterilisierung der Vorrichtung vereinfachen.

Eine Vorrichtung mit einem Körper aus einem elastischen Material, wie sie hier beschrieben ist, umfasst insbesondere einen ersten Balg und einen zweiten Balg, die in den Körper eingebettet sind, wobei die Anordnung des ersten Balgs und des zweiten Balgs in dem Körper im Wesentlichen der Anordnung und Ausdehnung des durch die Atmung bewegten Bereichs und des Herzens im Körper einer Mutter entspricht.

Der durch die Atmung bewegte Bereich des Körpers einer Mutter ist aufgrund der Elastizität des menschlichen Körpers größer als der von den Lungenflügeln eingenommene Bereich und erstreckt sich insbesondere aufgrund der Bewegung des Zwerchfells in den Bauchraum hinein. Durch die beschriebene Anordnung der Balgen kann eine Bewegung der Liegefläche erzeugt werden, die in guter Näherung der Bewegung der Vorderseite des Oberkörpers einer Mutter oder eines anderen erwachsenen Menschen durch Atmung und/oder Herzschlag entspricht. Durch die Ausgestaltung, insbesondere die Form bzw. Gestalt, der Liegefläche und eines Randbereichs an bzw. um die Liegefläche kann die Topologie der Vorderseite des Oberkörpers einer Mutter oder eines anderen erwachsenen Menschen simuliert werden. Beides kann dazu dienen, einem Kind, insbesondere einem Neugeborenen, eine Umgebung zu bieten, die derjenigen auf der Vorderseite des Oberkörpers der liegenden Mutter ähnelt.

Der erste Balg ist insbesondere zweiteilig bzw. paarig. Anders ausgedrückt sind insbesondere zwei erste Balge symmetrisch in dem Körper angeordnet, um die Lungenflügel des menschlichen Körpers zu simulieren.

Bei einer Vorrichtung mit einem Körper aus einem elastischen Material, wie sie hier beschrieben ist, sind insbesondere das Material des Körpers und ggf. das Material der Folie und ggf. das Material des oder der Balge für Röntgenstrahlung transparent.

Insbesondere weisen die Materialien der genannten Bestandteile der Vorrichtung keine Metalle oder andere Elemente mit höherer Ordnungszahl auf, die Röntgenstrahlung absorbieren oder streuen. Dies kann eine Röntgenaufnahme eines auf der Liegefläche der Vorrichtung liegenden Kinds durch den Körper hindurch ermöglichen.

Bei einer Vorrichtung mit einem Körper aus einem elastischen Material, wie sie hier beschrieben ist, sind insbesondere das Material des Körpers und ggf. das Material der Folie und ggf. das Material des oder der Balge für sichtbares Licht und/oder für ultraviolettes Licht transparent oder transluzent.

Eine transparente oder transluzente Ausgestaltung der Vorrichtung kann eine Fototherapie eines auf der Liegefläche der Vorrichtung liegenden Kinds auch von der Unterseite ermöglichen.

Eine Vorrichtung mit einem Körper aus einem elastischen Material, wie sie hier beschrieben ist, ist insbesondere für eine Verwendung in einem System, wie es hier beschrieben ist, oder für eine Kombination mit einer Vorrichtung zum Erfassen und Übertragen von von einer Bezugsperson ausgehenden Sinnesreizen vorgesehen und ausgebildet. Ein System umfasst insbesondere einee Vorrichtung mit einem Körper aus einem elastischen Material, wie sie hier beschrieben ist, und eine Vorrichtung zum Erfassen und Übertragen von von einer Bezugsperson ausgehenden Sinnesreizen.

Nachfolgend werden verschiedene Ausführungsformen anhand der beigefügten Figuren näher erläutert. Es zeigen:
- Figur 1: eine schematische Darstellung eines Systems;
- Figur 2: eine schematische axonometrische Darstellung eines Inkubators mit einer Vorrichtung zum Erzeugen von Sinnesreizen;
- Figur 3: eine schematische Explosionsansicht der Vorrichtung zum Erzeugen von Sinnesreizen;
- Figur 4: eine weitere schematische Darstellung der Vorrichtung aus den Figuren 2 und 3;
- Figur 5: eine schematische axonometrische Darstellung einer Vorrichtung zum Erfassen von Sinnesreizen;
- Figur 6: eine weitere schematische Darstellung der Vorrichtung aus Figur 5;
- Figur 7: eine weitere schematische Darstellung der Vorrichtungen aus den Figuren 5 und 6;
- Figur 8: eine weitere schematische Darstellung der Vorrichtung aus den Figuren 5 bis 7;
- Figur 9: eine schematische Darstellung einer Steuereinrichtung;
- Figur 10: ein schematisches Flussdiagramm;
- Figur 11: eine schematische Gegenüberstellung einer Mutter mit einer Vorrichtung zum Erzeugen von Sinnesreizen;
- Figur 12: eine schematische axonometrische Schnittdarstellung einer weiteren Vorrichtung zum Erzeugen von Sinnesreizen;
- Figur 13: eine weitere schematisch axonometrische Schnittdarstellung der Vorrichtung aus Figur 12
- Figur 14: eine schematische Draufsicht und zwei schematische Schnittdarstellungen der Vorrichtung aus den Figuren 12 und 13;
- Figur 15: eine weitere schematisch Schnittdarstellung der Vorrichtung aus den Figuren 12 bis 14.

Figur 1 zeigt eine schematische Darstellung eines Systems zum Übertragen von Sinnesreizen zwischen einer Mutter 20 und einem von der Mutter räumlich beabstandeten Säugling 21. Die Mutter 20 trägt eine Aufnehmereinheit 30 in einer ähnlichen Position, wie sie den Säugling 21 tragen könnte, wenn nicht beispielsweise medizinische Gründe dagegen sprächen. Die Aufnehmereinheit 30 ist mittels eines Tragegurts 31 an der Mutter 20 befestigt. Merkmale der Aufnehmereinheit 30 sind unten mit Bezug auf die Figuren 5 bis 8 näher beschrieben.

Der Säugling 21 liegt auf einer Matratze 60, die in einem Inkubator angeordnet sein kann. Die Matratze 60 weist eine Liegefläche 61 mit einem Fußende 62, einem Kopfende 63 und Längsseiten 65 auf. An den beiden einander gegenüberliegenden Längsseiten 65 der Liegefläche 61 sind je ein Hohlraum 68 mit jeweils mehreren Öffnungen 69 vorgesehen. Am Kopfende 63 der Liegefläche 61 sind ein oder mehrere Lautsprecher 71 vorgesehen, insbesondere hinter entsprechenden Öffnungen in der Oberfläche der Matratze 60. In der Nähe des Kopfendes 63 der Liegefläche 61 sind ein oder mehrere Mikrofone 75 vorgesehen. Insbesondere ist an jeder Längsseite 65 nahe dem Kopfende 63 der Matratze 60 und nahe dem Kopf des Säuglings 21 ein Mikrofon 75 angeordnet. Merkmale und Eigenschaften der Matratze 60 sind unten mit Bezug auf die Figuren 2 bis 4 näher beschrieben.

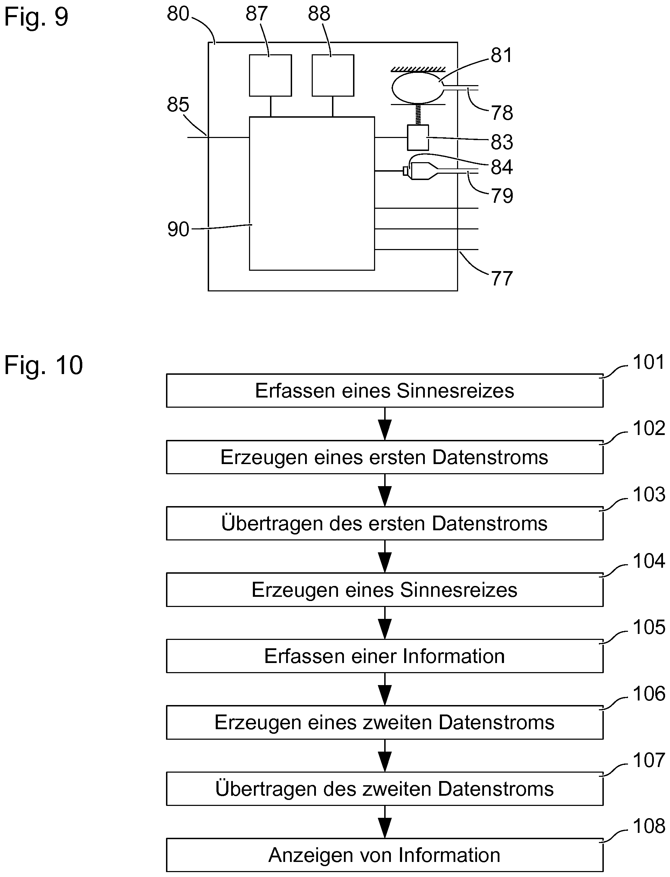

Die Matratze 60 ist über Signal- und Fluidleitungen 77, 78, 79, die in ein Bündel zusammengefasst sind, mit einer Steuereinrichtung 80 verbunden. Die Steuereinrichtung 80 ist unten mit Bezug auf Figur 9 näher beschrieben. Ein erster Datenstrom 27 wird von der Aufnehmereinheit 30 zur Steuereinrichtung 80 übertragen. Ein zweiter Datenstrom 28 wird von der Steuereinrichtung 80 zur Aufnehmereinheit 30 übertragen.

Figur 2 zeigt eine schematische axonometrische Darstellung der Matratze 60 in einem als einfacher transparenter Quader angedeuteten Inkubator 22. In der axonometrischen Darstellung der Figur 2 ist erkennbar, dass ein die Liegefläche 61 begrenzender umlaufender Randbereich 64 der Matratze 60 erhaben ausgebildet ist. Die Hohlräume 68 sind in mittleren Bereichen der Längsseiten 65 unter dem Randbereich 64 ausgebildet. Dazu ist der Randbereich 64 in den Mitten der Längsseiten 65 nach oben und zur Liegefläche 61 hin gewölbt.

Figur 3 zeigt eine weitere schematische axonometrische Darstellung der Matratze 60, wobei Bestandteile der Matratze 60 in der Art einer Explosionsdarstellung relativ zueinander vertikal verschoben sind. Die Matratze 60 umfasst ein Paar im Wesentlichen symmetrisch ausgebildeter und angeordneter erster Balge 72 und einen zweiten Balg 73 in einem hohlen Grundelement 74, der Oberseite die Liegefläche 61 bildet.

Die ersten Balge 72 sind mit einer ersten Fluidleitung 78 verbunden. Der zweite Balg 73 ist mit einer zweiten Fluidleitung 79 verbunden. Die ersten Balge 72 nehmen einen großen Teil der Fläche des Grundelements 74 und einen großen Teil der Liegefläche 61 ein. Durch Zu- und Abfuhr von Luft, einem anderen Gas oder einer Flüssigkeit zu bzw. von den ersten Balgen 72 kann die Liegefläche 61 großflächig vertikal bewegt bzw. gehoben und gesenkt werden. Durch Zu- und Abfuhr von Luft oder einem anderen Fluid durch die zweite Fluidleitung zu bzw. von dem zweiten Balg 73 kann die Liegefläche 61 ebenfalls, jedoch weniger großräumig gehoben bzw. gesenkt werden. Die ersten Balge 72 sind zum niederfrequenten Heben und Senken der Liegefläche 61 entsprechend den Atembewegungen des Brustkorbs der Mutter 20 vorgesehen und ausgebildet. Der zweite Balg 73 ist zur Erzeugung von Körperschall oder Vibrationen in der Liegefläche 61 - insbesondere entsprechend den Herztönen der Mutter 20 - vorgesehen und ausgebildet.

Figur 4 zeigt eine weitere schematische axonometrische Darstellung der Matratze 60. Die Matratze 60 ist teilweise aufgeschnitten darstellt, um strukturelle Merkmale sichtbar zu machen.

Bei dem hier dargestellten Beispiel ist das Grundelement einstückig ausgebildet und bilden eine Tasche, in der die ersten Balge 72 und der zweite Balg 73 angeordnet sind. Die Oberseite des Grundelements 74 bildet die Liegefläche 61. Der Randbereich 64 der Liegefläche ist als separates, lediglich lose aufgelegtes oder aber mit dem Rest der Matratze 60 verklebtes oder auf andere Weise gefügtes Bauteil ausgebildet. Der Hohlraum 68 ist durch einen außen umlaufenden Schlitz 76 zugänglich.

Figur 5 zeigt eine schematische axonometrische Darstellung der Aufnehmereinheit 30 aus Figur 1. Die Aufnehmereinheit 30 weist als Ösen ausgebildete Befestigungseinrichtungen 32 für Tragegurte 31 (vgl. Figur 1) auf. Die Aufnehmereinheit 30 weist ein erstes Mikrofon 41 und eine Anzeigeeinrichtung 45 an der Oberfläche auf, die in Figur 5 teilweise dem Betrachter zugewandt dargestellt und für eine von der Mutter 20 abgewandte Anordnung vorgesehen ist.

Die Anzeigeeinrichtung 45 ist ausgebildet, um durch Symbole, Piktogramme oder auf andere grafische Weise, alphanumerisch, akustisch und/oder mittels Lichtsignalen den Betriebszustand der Aufnehmereinheit 30 und/oder im zweiten Datenstrom 28 (vgl. Figur 1) enthaltene Information über den Säugling 21 darzustellen. Zu der darzustellenden Information über den Säugling 21 zählen insbesondere sein Gesundheitszustand, sein Wach- oder Schlafzustand, sein Puls, sein Blutdruck, die Sauerstoffsättigung seines Bluts und andere Parameter. Ferner kann die Aufnehmereinheit 30 ausgebildet sein, um vom Säugling 21 erzeugte und mittels des zweiten Datenstroms 28 übertragene Geräusche mittels eines Lautsprechers zu reproduzieren.

Die Figuren 6 bis 8 zeigen weitere schematische Darstellungen der Aufnehmereinheit aus den Figuren 1 und 5. In Figur 6 ist die bei der vorgesehenen Verwendung der Aufnehmereinheit 30 von der Mutter abgewandte Seite gezeigt. In Figur 8 ist die bei der vorgesehenen Verwendung der Aufnehmereinheit 30 der Mutter zugewandte Seite gezeigt. In Figur 7 ist die Aufnehmereinheit 30 von der Seite gezeigt, die Zeichenebene der Figur 7 ist also senkrecht zu den Zeichenebenen der Figuren 6 und 8.

In Figur 8 sind an der der Mutter zugewandten Seite ein Sensor 42 zum Erfassen von Bewegungen des Brustkorbs der Mutter und ein Mikrofon 43 zum Erfassen von Herztönen der Mutter vorgesehen. Der Sensor 42 und das zweite Mikrofon 43 sind insbesondere vorgesehen und ausgebildet, um unmittelbar an der Haut der Mutter anzuliegen. Der Sensor zum Erfassen von Bewegungen des Brustkorbs kann als Beschleunigungssensor ausgebildet sein und in diesem Fall im Inneren der Aufnehmereinheit 30 angeordnet sein. Ferner kann der Sensor zum Erfassen von Bewegungen des Brustkorbs in Form eines Kraftaufnehmers in die Befestigungseinrichtung 32 für einen Tragegurt 31 (vgl. Figur 1) oder in einen Tragegurt 31 integriert sein.

Im Inneren der Aufnehmereinheit 30 sind unsichtbar und deshalb in den Figuren 6 und 8 lediglich durch gestrichelte Linien angedeutet, eine Übertragungseinrichtung bzw. ein Sender 47 und eine Empfangseinrichtung bzw. ein Empfänger 48 angeordnet. Das erste Mikrofon 41, der Sensor 42 und das zweite Mikrofon 43 sind durch mit gestrichelten Linien angedeutete Signalleitungen mit dem Sender 47 verbunden. Die Anzeigeeinrichtung 45 ist durch eine mit gestrichelten Linien angedeutete Signalleitung mit dem Empfänger 48 verbunden.

Der Sender 47 und der Empfänger 48 können jeweils eine Datenverarbeitungseinrichtung aufweisen, beispielsweise zur Analog-Digital-Wandlung, zur Digital-Analog-Wandlung, zur Datenkompression oder zur Datendekompression. Alternativ oder zusätzlich können das erste Mikrofon 41, der Sensor 42, das zweite Mikrofon 43 und die Anzeigeeinrichtung 45 jeweils eine Datenverarbeitungseinrichtung aufweisen, insbesondere zur Datenkompression, zur Datendekompression, zur Analog-Digital-Wandlung oder zur Digital-Analog-Wandlung. Abweichend von der Darstellung in den Figuren 6 und 8 können das erste Mikrofon 41, der Sensor 42, das zweite Mikrofon 43, die Anzeigeeinrichtung 45, der Sender 47 und der Empfänger 48 teilweise oder vollständig integriert sein. Ferner kann abweichend von der Darstellung in den Figuren 6 und 8 eine separate Datenverarbeitungseinrichtung vorgesehen sein, die mit dem ersten Mikrofon 41, dem Sensor 42, dem zweiten Mikrofon 43, der Anzeigeeinrichtung 45, dem Sender 47 und dem Empfänger 48 durch Signalleitungen verbunden ist.

In der Zusammenschau der Figuren 1 und 5 bis 8 ist erkennbar, dass die Aufnehmereinheit 30 eine Form bzw. Gestalt aufweist, die einerseits ergonomischen Erfordernissen entspricht und andererseits Ähnlichkeit mit der Gestalt des Rumpfs eines Säuglings aufweist. Insbesondere weist die Aufnehmereinheit 30 an der der Mutter zugewandten Seite große Krümmungsradien und eine weiche Oberfläche auf.

Die Aufnehmereinheit 30 ist eine Vorrichtung zum Erfassen und Übertragen von von der Mutter 20 oder einer anderen Bezugsperson ausgehenden bzw. erzeugten Sinnesreizen. Eine solche Vorrichtung kann abweichend von den Eigenschaften der hier dargestellten Aufnehmereinheit 30 mehrere separate Bestandteile aufweisen. Diese Bestandteile sind insbesondere durch Kabel oder eine andere Signalstrecke (insbesondere Bluetooth oder einem anderen Funkstandard entsprechend) miteinander gekoppelt. Beispielsweise sind Sensoren in einer oder mehreren Einheiten am Brustkorb angeordnet, deren Signale in analoger oder digitaler Form an eine separate Datenverarbeitungseinheit übermittelt werden. Die separate Datenverarbeitungseinheit kann beispielsweise am Gürtel oder in einer Tasche getragen werden und Kommunikationsmittel zur Übertragung des ersten Datenstroms per WLAN, WiFi, GSM, UMTS oder auf andere Weise umfassen.

Figur 9 zeigt eine schematische Darstellung der Steuereinrichtung 80 aus Figur 1. Die Steuereinrichtung 80 umfasst einen Steuerbalg 81, der mittels der ersten Fluidleitung 78 mit den ersten Balgen 72 unter der Liegefläche 61 (vgl. Figuren 3 und 4) verbunden ist. Das Volumen des ersten Steuerbalgs 81 ist durch einen zugeordneten Antrieb 83 veränderbar. Der Antrieb 83 umfasst beispielsweise einen Schrittmotor oder einen anderen Elektromotor und eine Gewindespindel oder ein anderes Getriebe.

Ferner umfasst die Steuereinrichtung 80 eine Einrichtung 84, die mittels der zweiten Fluidleitung 79 mit dem zweiten Balg 73 (vgl. Figuren 3 und 4) fluidisch gekoppelt ist. Die Einrichtung 84 umfasst beispielsweise eine durch einen Elektromagneten angetriebene Membran, mittels derer ein Volumen variiert werden kann, das mittels der zweiten Fluidleitung 79 mit dem zweiten Balg 73 verbunden werden kann. Die Membran und der Elektromagnet können Bestandteil eines herkömmlichen Lautsprechers oder in der Art eines herkömmlichen Lautsprechers angeordnet sein. Alternativ ist die Einrichtung 84 eine Pendelpumpe oder eine andere Einrichtung zur gesteuerten Variation des Drucks in einem Fluid.

Ferner umfasst die Steuereinrichtung 80 eine Empfangseinrichtung bzw. einen Empfänger 87 für den ersten Datenstrom 27 (vgl. Figur 1) und eine Übertragungseinrichtung bzw. einen Sender 88 für den zweiten Datenstrom 28. Der Empfänger 87 und der Sender 88 können abweichend von der Darstellung in Figur 9 teilweise oder vollständig in ein gemeinsames Bauteil integriert sein. Der Sender 47 und der Empfänger 48 sind jeweils zum Senden bzw. Empfangen von elektrischen Signalen über eine oder mehrere elektrische Leitungen, von elektromagnetischen Signalen mittels einer oder mehrerer Antennen und/oder von (sichtbaren oder sichtbaren) Lichtsignalen mittels Lichtquellen bzw. Lichtsensoren ausgebildet. Der Sender 47 und der Empfänger 48 entsprechen beispielsweise einem Industriestandard, beispielsweise Bluetooth, WLAN, GSM, UMTS bzw. 3G, DECT.

Ferner umfasst die Steuereinrichtung 80 eine Datenverarbeitungseinrichtung 90. Die Datenverarbeitungseinrichtung 90 kann einen oder mehrere Prozessoren und/oder Controller, flüchtigen und nicht-flüchtigen Speicher und weitere Bestandteile enthalten. Die Datenverarbeitungseinrichtung 90 ist mit dem Antrieb 83 für den Steuerbalg 81, mit der Einrichtung 84, mit dem Empfänger 87 und mit dem Sender 88 wirksam verbunden. Ferner ist die Datenverarbeitungseinrichtung 90 mittels Signalleitungen 77 mit den Mikrofonen 75 und dem Lautsprecher 71 (vgl. Figur 1) verbunden.

Figur 10 zeigt ein schematisches Flussdiagramm eines Verfahrens zum Erzeugen von Sinnesreizen, die den von einer Bezugsperson ausgehenden Sinnesreizen entsprechen, für einen von der Bezugsperson räumlich beabstandeten Säugling oder ein anderes von der Bezugsperson räumlich beabstandetes Kind. Das Verfahren ist insbesondere ausführbar mit einem System und Vorrichtungen mit Eigenschaften und Merkmalen, wie sie oben anhand der Figuren 1 bis 9 dargestellt sind. Ferner ist das Verfahren jedoch auch mit einem System und Vorrichtungen ausführbar, die von den oben anhand der Figuren 1 bis 9 dargestellten Merkmalen und Eigenschaften abweichende Merkmale und Eigenschaften aufweisen. Trotzdem wird nachfolgend beispielhaft auf das System und die Vorrichtungen Bezug genommen, die oben anhand der Figuren 1 bis 9 dargestellt sind, um das Verständnis zu vereinfachen.

Bei einem ersten Schritt 101 werden mittels eines Mikrofons 43 an einer von einer Bezugsperson 20 getragenen Aufnehmereinheit 30 Herztöne der Bezugsperson oder andere von der Bezugsperson erzeugte und sich im Wesentlichen in Form von Körperschall im Rumpf der Bezugsperson 20 ausbreitende Geräusche oder Vibrationen erfasst. Alternativ oder zusätzlich können beim ersten Schritt 101 Bewegungen des Brustkorbs der Bezugsperson 20 (insbesondere Atembewegungen) oder andere Bewegungen der Bezugsperson 20 mittels eines Sensors 42 an der Aufnehmereinheit 30 erfasst werden. Alternativ oder zusätzlich kann beim ersten Schritt 101 ein von der Bezugsperson 20 erzeugter Luftschall mittels eines Mikrofons 41 erfasst werden, insbesondere Gesprochenes oder Gesungenes. Alternativ oder zusätzlich können beim ersten Schritt 101 die Körpertemperatur der Bezugsperson 20 erfasst werden. Alternativ oder zusätzlich können beim ersten Schritt 101 Eingaben der Bezugsperson 20 an einer Tastatur oder einer anderen Benutzerschnittstelle erfasst werden. Alle beim ersten Schritt 101 erfassten Parameter und Daten können ursprünglich insbesondere in Form analoger elektrischer Signale vorliegen, die anschließend vor oder bei dem nachfolgend beschriebenen zweiten Schritt digitalisiert werden können.

Bei einem zweiten Schritt 102 wird ein erster Datenstrom 27 erzeugt. Der erste Datenstrom 27 ist insbesondere ein kontinuierlich erzeugter Datenstrom, der in analoger oder digitaler Form mehrere oder alle der beim ersten Schritt 101 erfassten Parameter enthält bzw. beschreibt. Beim zweiten Schritt kann eine Auswahl oder eine Manipulation von Daten stattfinden. Insbesondere können Daten gefiltert, unterdrückt bzw. ausgeblendet oder ersetzt werden. Beispielsweise hat die Bezugsperson 20 die Möglichkeit mittels einer Taste oder eines Schalters an der Aufnehmereinheit 30 ein Mikrofon auszuschalten oder die Empfindlichkeit eines Mikrofons zu reduzieren, wenn sich die Bezugsperson in einer lauten Umgebung befindet oder unangenehmen Geräuschen ausgesetzt ist.

Bei einem dritten Schritt 103 wird der erste Datenstrom 27 von der Aufnehmereinheit 30, insbesondere von einem Sender 47 der Aufnehmereinheit 30, zu einer räumlich beabstandeten Steuereinrichtung 80, insbesondere zu einem Empfänger 87 der Steuereinrichtung 80 übertragen. Die Übertragung des ersten Datenstroms kann in Form elektrischer, elektromagnetischer, akustischer oder anderer Signale erfolgen. Dabei wird der erste Datenstrom 27 insbesondere durch eine Sendeeinrichtung bzw. einen Sender 47 der Aufnehmereinheit 30 gesendet und durch eine Empfangseinrichtung bzw. einen Empfänger 87 der Steuereinrichtung 80 empfangen.

Bei einem vierten Schritt 104 werden gesteuert durch den ersten Datenstrom 27 von der Steuereinrichtung 80 und einer Matratze 60 Sinnesreize für ein Kind 21 erzeugt, die den beim ersten Schritt 101 erfassten Sinnesreizen entsprechen oder im Wesentlichen entsprechen. Insbesondere werden gesteuert durch eine Datenverarbeitungseinrichtung 90 der Steuereinrichtung 80 ein Antrieb 83 für einen Steuerbalg 81 gesteuert und damit das Volumen des Steuerbalgs 81 variiert, wobei aufgrund der fluidischen Kopplung des Steuerbalgs 81 mit ersten Balgen 72 eine Liegefläche 61 einer Matratze 60 entsprechend den beim ersten Schritt 101 erfassten Atembewegungen der Bezugsperson 20 gehoben und gesenkt wird. Alternativ oder zusätzlich wird beim vierten Schritt 104 mittels einer Einrichtung 84 der Steuereinrichtung 80, einer zweiten Fluidleitung 79 und eines zweiten Balgs 73, der mit der Liegefläche 61 mechanisch gekoppelt ist, Körperschall oder eine Vibration in der Liegefläche 71 erzeugt, der insbesondere von der Bezugsperson 20 erzeugten und beim ersten Schritt 101 erfassten Herztönen entspricht. Alternativ oder zusätzlich wird beim vierten Schritt 104 durch einen oder mehrere Lautsprecher 71 Luftschall erzeugt, der durch die Bezugsperson 20 erzeugtem und beim ersten Schritt 101 mittels des Mikrofons 41 erfasstem Luftschall entspricht.

Bei einem fünften Schritt 105 wird zumindest entweder Schall, der von dem Kind 21 erzeugt wird oder eine Bewegung des Kinds 21 erfasst. Vom Kind 21 erzeugter Schall wird insbesondere durch ein oder mehrere Mikrofone 75 an der Matratze 60 erfasst. Bewegungen des Kinds können optisch, mittels Dehnungsmessstreifen oder anderer Kraftsensoren oder mittels Beschleunigungssensoren erfasst werden. Ferner können beim fünften Schritt 105 alternativ oder zusätzlich die Körpertemperatur, der Puls, der Blutdruck, der Sauerstoffgehalt im Blut und/oder andere Parameter, die den Gesundheitszustand und/oder den Schlaf- oder Wachzustand des Kinds 21 beschreiben oder charakterisieren, erfasst werden. Diese Parameter können durch Sensoren erfasst werden, die Bestandteil des hier beschriebenen Systems, insbesondere der Matratze 60 sind. Alternativ können die Parameter von anderen medizinischen Instrumenten empfangen werden, insbesondere über einen Signaleingang 85 an der Steuereinrichtung 80.

Bei einem sechsten Schritt 106 wird ein zweiter Datenstrom 28 erzeugt, der den beim fünften Schritt 105 erfassten Schall und/oder die beim fünften Schritt 105 erfasste Bewegung des Kinds und/oder weitere beim fünften Schritt 105 erfasste Parameter und Information beschreibt. Der zweite Datenstrom 28 kann den Schall und/oder die Bewegung und ggf. den oder die weiteren Parameter und Informationen in analoger oder digitaler Weise beschreiben. Der sechste Schritt 106 wird insbesondere durch die Steuereinrichtung 80, beispielsweise durch eine Datenverarbeitungseinrichtung 90 der Steuereinrichtung 80, ausgeführt.

Bei einem siebten Schritt 107 wird der zweite Datenstrom 28 von der Steuereinrichtung 80 zur Aufnehmereinheit 30 übertragen. Dies erfolgt in Form elektrischer, elektromagnetischer, Licht-, akustischer oder anderer Signale. Dabei wird der zweite Datenstrom 28 insbesondere durch eine Sendeeinrichtung bzw. einen Sender 88 der Steuereinrichtung 80 gesendet und durch eine Empfangseinrichtung bzw. einen Empfänger 48 der Aufnehmereinheit 30 empfangen.

Bei einem achten Schritt 108 wird der zweite Datenstrom 28 in durch den zweiten Datenstrom 28 beschriebenen Schall und/oder in eine durch den zweiten Datenstrom 28 beschriebene Bewegung gewandelt. Alternativ oder zusätzlich wird beim achten Schritt 108 ein durch den zweiten Datenstrom 28 beschriebener Zustand des Kinds 21 in Form von Symbolen oder Piktogrammen oder auf andere grafische, alphanumerische, akustische Weise oder mittels Lichtzeichen angezeigt. Der achte Schritt 108 wird insbesondere durch die Anzeigeeinrichtung 45 an der Aufnehmereinheit 30 ausgeführt.

Alle Schritte werden insbesondere kontinuierlich oder quasikontinuierlich und dabei im Wesentlichen gleichzeitig ausgeführt. Insbesondere der fünfte Schritt 105, der sechste Schritt 106, der siebte Schritt 107 und der achte Schritt 108 sind optional.

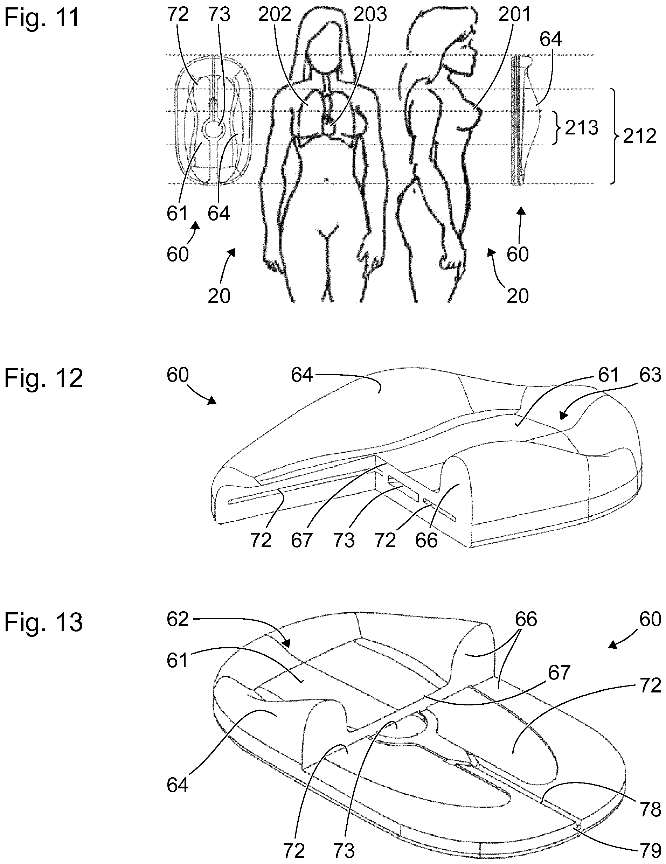

Figur 11 zeigt eine schematische Gegenüberstellung einer Mutter 20 mit einer Matratze 60, wie sie oben anhand der Figuren 1 bis 4 dargestellt ist. Die Mutter 20 mit durchschnittlicher Körpergröße und durchschnittlichem Körperbau im jüngeren gebärfähigen Alter und die Matratze 60 sind näherungsweise im gleichen Maßstab dargestellt. In Figur 11 ist in der Mitte die Mutter 20 zweimal, nämlich links frontal und rechts von der Seite dargestellt. Auch die Matratze 60 ist zweimal dargestellt, nämlich links in Draufsicht und rechts in einer Seitenansicht.

In der Frontalansicht der Mutter 20 sind die Positionen und Ausdehnungen von Lungenflügeln 202 und Herz 203 angedeutet. In der Draufsicht der Matratze 60 sind der erste Balg 72 zum Erzeugen von vertikalen Bewegungen (senkrecht zur Zeichenebene der Figur 11) der Liegefläche 61 der Matratze 60 und der zweite Balg 73 zum Erzeugen von Körperschall, der insbesondere dem des menschlichen Herzschlags entspricht, in der Matratze 60 angedeutet.

Durch horizontale gestrichelte Linien sind in Figur 11 der durch die Atmung bewegte Bereich 212 des Oberkörpers der Mutter 20 und der Bereich 213, in dem das Herz 203 der Mutter 20 liegt, angedeutet. Der durch die Atmung bewegte Bereich 212 des Oberkörpers der Mutter 20 ist etwas größer als der von den Lungenflügeln 202 eingenommene Bereich und erstreckt sich bis in den Bauchraum der Mutter 20, da durch die Bewegung des Zwerchfells bei der Atmung auch der Bauchraum und die darin befindlichen Organe bewegt werden.

In der in Figur 11 dargestellten Gegenüberstellung der Mutter 20 und der Matratze 60 ist erkennbar, dass die Ausdehnung der ersten Balge 72 in der Matratze 60 im Wesentlichen dem durch die Atmung bewegten Bereich 212 des Oberkörpers der Mutter 20 und der Bereich, innerhalb dessen der zweite Balg 73 angeordnet ist, im Wesentlichen dem Bereich 213, in dem das Herz 203 der Mutter 20 angeordnet ist, entsprechen. Ferner ist erkennbar, dass der umlaufende Randbereich 64 der Matratze 60 in einem mittleren seitlichen Bereich näherungsweise entsprechend den Brüsten 201 der Mutter 20 erhöht und verbreitert ist.

Durch die Anordnung und die Gestalt der ersten Balge 72 und des zweiten Balgs 73 in der Matratze 60, die Form bzw. Gestalt der Liegefläche 61 und die Form bzw. Gestalt des umlaufenden Randbereichs 64 der Matratze 60 wird somit die Topologie der Vorderseite des Oberkörpers der Mutter 20 und von deren Bewegung durch die Atmung und Herzschlag simuliert.

Figur 12 zeigt eine schematische axonometrische Schnittdarstellung einer weiteren als Vorrichtung zum Erzeugen von Sinnesreizen für ein Kind ausgestalteten Matratze 60, die in einigen Merkmalen und Eigenschaften, insbesondere in der Form bzw. Gestalt der Liegefläche 61 und des umlaufenden Randbereichs 64 der Matratze 60, den oben anhand der Figuren 1 bis 4 und 11 dargestellten Matratzen ähnelt. Nachfolgend sind lediglich Merkmale und Eigenschaften beschrieben, in denen sich die Matratze 60 von den oben anhand der Figuren 1 bis 4 und 11 dargestellten Matratzen unterscheidet. Die Matratze 60 ist durch zwei Schnitte in zwei vertikalen und zueinander orthogonalen Halbebenen aufgeschnitten dargestellt, um den inneren Aufbau der Matratze 60 zu zeigen.

Die Matratze 60 unterscheidet sich von den oben anhand der Figuren 1 bis 4 und 11 dargestellten Matratzen insbesondere dadurch, dass die Balge 72, 73 nicht in einem Hohlraum unter der Liegefläche angeordnet, sondern in einen Körper 66 aus einem elastischen Material eingebettet sind. Der Körper 66 besteht insbesondere aus Polyurethan-Gummi, Polyurethan-Gel, Latex oder einem anderen elastischen Material. Das elastische Material des Körpers 66 weist insbesondere eine ähnliche Massendichte und eine ähnliche Elastizität wie menschliches Muskel- oder Fettgewebe auf. Das elastische Material des Körpers 66 weist insbesondere eine möglichst hohe Wärmeleitfähigkeit auf, um eine gleichmäßige Temperierung innerhalb eines Inkubators 22 (vgl. Figur 2) zu ermöglichen.

An der Oberfläche des Körpers 66 ist eine elastische Folie 67 vorgesehen, die insbesondere die Liegefläche 61 der Matratze 60 bildet. Die elastische Folie 67 kann den Körper 66 bzw. dessen Oberfläche vollständig bedecken. Alternativ kann beispielsweise die von der Liegefläche 61 abgewandte Unterseite der Matratze 60 durch eine steifere Folie bzw. eine Folie geringerer Elastizität bedeckt sein. Die Folie 67 weist insbesondere eine glatte, abwischbare, geschlossene Oberfläche auf, die eine Reinigung und Sterilisierung der Matratze 60 vereinfacht. Insbesondere ist die elastische Folie 67 ausgebildet, um eine geringe Durchlässigkeit für Keime aufzuweisen.

Die Balge 72, 73 sind insbesondere vollständig und unmittelbar vom elastischen Material des Körpers 66 umgeben. Die Matratze 60 weist innerhalb des durch die Folie 67 umschlossenen Raumbereiches außer den Balgen 72, 73 keine weiteren Hohlräume oder Öffnungen auf, in denen sich Keime ansiedeln könnten.

Das elastische Material des Körpers 66, das Material der elastischen Folie 67 und ggf. die Materialien elastischer Folien, die die Oberflächen der Balge 72, 73 bilden, sind insbesondere für Röntgenstrahlung transparent, um Röntgenaufnahmen eines Kinds auf der Liegefläche 61 durch die Matratze 60 hindurch zu ermöglichen. Dazu weisen das elastische Material des Körpers 66, das Material der elastischen Folie 67 und ggf. die Materialien von Folien, die die Oberflächen der Balgen 72, 73 bilden, insbesondere ausschließlich leichte Elemente bzw. Elemente mit niedriger Ordnungszahl auf, die Röntgenstrahlung nicht oder nur im geringen Maße absorbieren oder streuen.

Das elastische Material des Körpers 66, das Material der elastischen Folie 67 und ggf. die Materialien elastischer Folien, die die Oberflächen der Balge 72, 73 bilden, sind insbesondere für sichtbares Licht und/oder ultraviolettes Licht transparent oder transluzent, um eine Fototherapie für ein auf der Liegefläche 61 der Matratze 60 liegendes Kind auch von der Unterseite zu ermöglichen.

Figur 13 zeigt eine weitere schematische axonometrische Schnittdarstellung der Matratze 60 aus Figur 12. Die Darstellung von Figur 13 unterscheidet sich von der Darstellung in Figur 12 dadurch, dass die Matratze entlang einer horizontalen Halbebene und einer vertikalen Halbebene aufgeschnitten ist. Aufgrund der Anordnung des horizontalen Schnitts ist die Gestalt der Balgen 72, 73 erkennbar, die denen der oben anhand der Figuren 1 bis 4 und 11 dargestellten Matratze entsprechen. Ferner sind ebenfalls in das elastische Material des Körpers 66 eingebettete Fluidleitungen 78, 78 zur fluidischen Ansteuerungen der Balgen 72, 73 erkennbar.

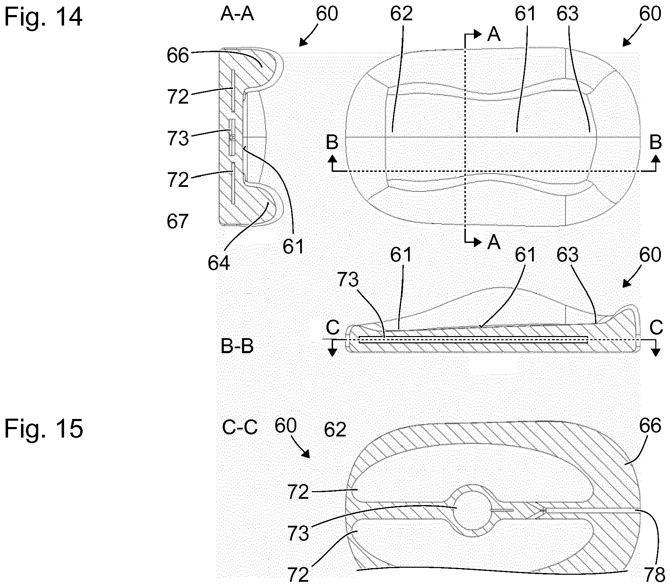

Figur 14 zeigt rechts oben eine schematische Draufsicht und links und unten zwei schematische Schnittdarstellungen der oben anhand der Figuren 12 und 13 dargestellten Matratze 60. Die Zeichenebene der Draufsicht rechts oben in Figur 14 ist horizontal. Die Schnittebene A-A des in Figur 14 links dargestellten Schnitts und die Schnittebene B-B des in Figur 14 unten dargestellten Schnitts sind in der in Figur 14 rechts oben dargestellten Draufsicht angedeutet. Die Schnittebene A-A ist so gewählt, dass sie nicht nur die ersten Balgen 72, sondern auch den zweiten Balg 73 schneidet. Die Schnittebene B-B ist so gewählt, dass sie lediglich einen der ersten Balgen 72 schneidet.

Figur 15 zeigt eine weitere schematische Schnittdarstellung der oben anhand der Figuren 12 bis 14 dargestellten Matratze 60. Die horizontale Schnittebene C-C der Figur 15 ist in dem in Figur 14 unten dargestellten Schnitt entlang der Ebene B-B angedeutet. Die Schnittebene C-C der Figur 15 verläuft innerhalb der Balgen 72, 73. Da die Matratze 14 spiegelsymmetrisch ausgestaltet ist, ist nur eine Hälfte vollständig, die andere unvollständig dargestellt.

In den Figuren 12 und 14 ist erkennbar, dass die Balgen 72, 73 jeweils flache Querschnitte aufweisen. In Figuren 13 und 15 ist die Erstreckung der Balgen 72, 73 in der horizontalen Ebene erkennbar.

### Bezugszeichenliste

- 10: System zum Übertragen von Sinnesreizen zwischen Mutter und Kind
- 20: Mutter
- 21: Säugling
- 22: Inkubator
- 27: erster Datenstrom von Mutter 20 zu Säugling 21
- 28: zweiter Datenstrom von Säugling 21 zur Mutter 20
- 30: Aufnehmereinheit
- 31: Tragegurt der Aufnehmereinheit 30
- 32: Befestigungseinrichtung für Tragegurt
- 41: erstes Mikrofon an der Aufnehmereinheit 30
- 42: Sensor an der Aufnehmereinheit 30 für Bewegungen des Brustkorbs der Mutter 20
- 43: zweites Mikrofon an der Aufnehmereinheit 30
- 45: Anzeigeeinrichtung an der Aufnehmereinheit 30
- 47: Sender der Aufnehmereinheit 30 für ersten Datenstrom 27
- 48: Empfänger der Aufnehmereinheit 30 für zweiten Datenstrom 28
- 60: Matratze
- 61: Liegefläche der Matratze 60
- 62: Fußende der Liegefläche 61
- 63: Kopfende der Liegefläche 61
- 64: umlaufender Randbereich der Matratze 60
- 65: Längsseite der Liegefläche 61
- 66: Körper aus einem elastischen Material
- 67: elastische Folie auf der Oberfläche des Körpers 66
- 68: Hohlraum für Geruchsquelle
- 69: Öffnung zum Hohlraum 68
- 71: Lautsprecher in der Matratze 60
- 72: erster Balg zum Erzeugen von vertikalen Bewegungen der Liegefläche 61
- 73: zweiter Balg zum Erzeugen von Körperschall in der Matratze 60
- 74: hohles Grundelement der Matratze 60
- 75: Mikrofon an der Matratze 60
- 76: außen umlaufender Schlitz
- 77: Signalleitung
- 78: erste Fluidleitung zwischen Matratze 60 und Steuereinrichtung 80
- 79: zweite Fluidleitung zwischen Matratze 60 und Steuereinrichtung 80
- 80: Steuereinrichtung
- 81: Steuerbalg der Fluidsteuereinrichtung 80 für ersten Balg 72
- 83: Antrieb für ersten Steuerbalg 81
- 84: Einrichtung
- 85: Signaleingang an der Steuereinrichtung 80
- 87: Empfänger für ersten Datenstrom 27
- 88: Sender für zweiten Datenstrom 28
- 90: Datenverarbeitungseinrichtung der Steuereinrichtung 80
- 101: erster Schritt (Erfassen)
- 102: zweiter Schritt (Erzeugen eines ersten Datenstroms)
- 103: dritter Schritt (Übertragen des ersten Datenstroms)
- 104: vierter Schritt (Wandeln des ersten Datenstroms)
- 105: fünfter Schritt (Erfassen)
- 106: sechster Schritt (Erzeugen eines zweiten Datenstroms)
- 107: siebter Schritt (Übertragen des zweiten Datenstroms)
- 108: achter Schritt (Wandeln des zweiten Datenstroms)
- 201: Brust der Mutter 20
- 202: Lungenflügel der Mutter 20
- 203: Herz der Mutter 20
- 212: durch die Atmung bewegter Bereich des Oberkörpers einer Mutter 20
- 213: Bereich, in dem das Herz 203 der Mutter 20 angeordnet ist

## Patentansprüche

1. **Vorrichtung** (60) zum Erzeugen von Sinnesreizen für ein Kind (21), mit:
einem **Körper** (66) aus einem elastischen Material;
einer **Liegefläche** (61) für ein Kind (21) auf dem Körper (66);
einem **Balg** (72, 73) zum Erzeugen einer vertikalen Bewegung der Liegefläche (61),
wobei der Balg (72, 73) **in das elastische Material des Körpers** (66) **eingebettet** ist,
**dadurch gekennzeichnet, dass** die Vorrichtung (60) durch ein **Gießverfahren** hergestellt ist, bei dem das elastische Material des Körpers (66) mit dem Balg (72) vollflächig oder im Wesentlichen vollflächig stoffschlüssig verbunden wird.

2. Vorrichtung (60) nach dem vorangehenden Anspruch, bei der der Körper (66) mit der Liegefläche (61) und dem Balg (72, 73) eine **Matratze** zur Anordnung in einem **Inkubator** (22) bildet.

3. Vorrichtung (60) nach einem der vorangehenden Ansprüche, bei der der Balg (72, 73) zumindest entweder zur Erzeugung einer vertikalen Bewegung, die einer durch die Atmung bedingten Bewegung der Vorderseite des Oberkörpers einer Mutter (20) oder eines anderen erwachsenen Menschen entspricht, oder zur Erzeugung von Körperschall, der dem Herzschlag einer Mutter (20) oder eines anderen erwachsenen Menschen entspricht, in dem elastischen Körper (66) vorgesehen und ausgebildet ist.

4. Vorrichtung (60) nach einem der vorangehenden Ansprüche, bei der der Körper (66) von dem Balg (72, 73) oder von mehreren Balgen (72, 73) abgesehen **keine weiteren Hohlräume** aufweist.

5. Vorrichtung (60) nach einem der vorangehenden Ansprüche, ferner mit:
einer **elastische Folie** (67) an der Oberfläche des Körpers (66), die zumindest die Liegefläche (61) bildet.

6. Vorrichtung (60) nach einem der vorangehenden Ansprüche, wobei bei dem Gießverfahren das elastische Material des Körpers (66) mit der elastischen Folie (67) vollflächig oder im Wesentlichen vollflächig stoffschlüssig verbunden wird.

7. Vorrichtung (60) nach einem der vorangehenden Ansprüche, mit:
einem **ersten Balg** (72) und einen **zweiten Balg** (73), die in den Körper (66) eingebettet sind,
wobei die Anordnung des ersten Balgs (72) und des zweiten Balgs (73) in dem Körper (66) im Wesentlichen der Anordnung und Ausdehnung des durch die **Atmung** bewegten Bereichs und des **Herzens** im Körper einer Mutter (20) entspricht.

8. Vorrichtung (60) nach einem der vorangehenden Ansprüche, bei der zwei **Balge** (72) **symmetrisch in dem Körper (66) angeordnet** sind, um die Lungenflügel des menschlichen Körpers (20) zu simulieren.

9. Vorrichtung (60) nach einem der vorangehenden Ansprüche, bei der zumindest entweder das Material des Körpers (66) oder das Material der elastischen Folie (67) oder das Material des Balgs (72) oder der Balgen (72, 73) **für Röntgenstrahlung transparent** ist.

10. Vorrichtung (60) nach einem der vorangehenden Ansprüche, bei der zumindest entweder das Material des Körpers (66) oder das Material der elastischen Folie (67) oder das Material des Balgs (72) oder der Balgen (72, 73) zumindest entweder **für sichtbares** Licht oder für **ultraviolettes** Licht **transparent oder transluzent** ist.

## Claims

1. **Device** (60) for generating sensory stimuli for a child (21), comprising:
a **body** (66) made of an elastic material;
a **lying area** (61) for a child (21) on the body (66);
a **bladder** (72, 73) for generating a vertical movement of the lying area (61),
wherein the bladder (72, 73) is **embedded in the elastic material of the body** (66),
**characterized in that**
the device (60) is produced by a **casting process,** in which the elastic material of the body (66) is connected by material bonding to the bladder (72) over the entire surface of the bladder (72) or essentially over the entire surface of the bladder (72).

2. Device (60) according to the preceding claim, wherein the body (66) with the lying area (61) and the bladder (72, 73) forms a **mattress** for placement in an **incubator** (22).

3. Device (60) according to one of the preceding claims, wherein the bladder (72, 73) is provided and configured for at least one of creating a vertical movement corresponding to the respiratory movement of the front side of the upper body of the mother (20) or of another adult person, and creating in the elastic body (66) body-borne sound that corresponds to the heartbeat of a mother (20) or of another adult person.

4. Device (60) according to one of the preceding claims, wherein the body (66) has **no further hollow spaces** apart from the bladder (72, 73) or the plurality of bladders (72, 73).

5. Device (60) according to one of the preceding claims, further comprising:
an **elastic film** (67) at the surface of the body (66), which forms at least the lying area (61).

6. Device (60) according to one of the preceding claims, wherein in the casting process the elastic material of the body (66) is connected by material bonding to the elastic film (67) over the entire surface of the film (67) or essentially over the entire surface of the film (67).

7. Device (60) according to one of the preceding claims, comprising:
a **first bladder** (72) and a **second bladder** (73) embedded in the body (66),
wherein the positions of the first bladder (72) and the second bladder (73) in the body (66) essentially correspond to the position and expansion of the area of a body of a mother (20) that is moved by breathing and of the heart.

8. Device (60) according to one of the preceding claims, wherein two **bladders** (72) **are arranged symmetrically in the body** (66) to simulate the lungs of the human body (20).

9. Device (60) according to one of the preceding claims, wherein at least one of the material of the body (66) and the material of the elastic film (67) and the material of the bladder (72) or of the bladders (72, 73) is **transparent** to x-rays.

10. Device (60) according to one of the preceding claims, wherein at least one of the material of the body (66) and the material of the elastic film (67) and the material of the bladder (72) or of the bladders (72, 73) is **transparent or translucent** to **visible** light or to **ultraviolet** light.

## Revendications

1. **Dispositif** (60) pour générer des stimuli sensoriels pour un enfant (21), comprenant :
un **corps** (66) en matériau élastique ;
une **surface de couchage** (61) pour un enfant (21) placée sur le corps (66) ;
un **soufflet** (72, 73) pour produire un mouvement vertical de la surface de couchage (61),
le soufflet (72, 73) étant **intégré dans le matériau élastique du corps** (66),
**caractérisé en ce que**
le dispositif (60) est fabriqué par un **procédé de moulage** dans lequel le matériau élastique du corps (66) est relié au soufflet (72) par liaison de matière sur toute la surface ou sensiblement sur toute la surface.

2. Dispositif (60) selon la revendication précédente, dans lequel le corps (66) avec la surface de couchage (61) et le soufflet (72, 73) forment un **matelas** prévu pour être placé dans une **couveuse** (22).

3. Dispositif (60) selon l'une des revendications précédentes, dans lequel le soufflet (72, 73) est prévu et réalisé dans le corps élastique (66) au moins soit pour générer un mouvement vertical correspondant à un mouvement de la face avant du torse d'une mère (20), ou d'une autre personne adulte, provoqué par la respiration, soit pour générer un bruit corporel correspondant au battement de cœur d'une mère (20) ou d'une autre personne adulte.

4. Dispositif (60) selon l'une des revendications précédentes, dans lequel le corps (66), à l'exception du soufflet (72, 73) ou de plusieurs soufflets (72, 73), **ne présente pas d'autres cavités.**

5. Dispositif (60) selon l'une des revendications précédentes, comprenant en outre :
un **film élastique** (67) à la surface du corps (66) formant au moins la surface de couchage (61).

6. Dispositif (60) selon l'une des revendications précédentes, dans lequel, lors du procédé de moulage, le matériau élastique du corps (66) est relié au film élastique (67) par liaison de matière sur toute la surface ou sensiblement sur toute la surface.

7. Dispositif (60) selon l'une des revendications précédentes, comprenant :
un premier soufflet (72) et un second soufflet (73) intégrés dans le corps (66),
la disposition du premier soufflet (72) et du second soufflet (73) dans le corps (66) correspondant sensiblement à la disposition et à l'extension de la région déplacée par la respiration et du cœur dans le corps d'une mère (20).

8. Dispositif (60) selon l'une des revendications précédentes, dans lequel deux **soufflets** (72) **sont disposés symétriquement dans le corps** (66) pour simuler les poumons du corps humain (20).

9. Dispositif (60) selon l'une des revendications précédentes, dans lequel au moins soit le matériau du corps (66), soit le matériau du film élastique (67), soit le matériau du soufflet (72) ou des soufflets (72, 73) est **transparent** aux rayons X

10. Dispositif (60) selon l'une des revendications précédentes, dans lequel au moins soit le matériau du corps (66), soit le matériau du film élastique (67), soit le matériau du soufflet (72) ou des soufflets (72, 73) est **transparent** ou **translucide** au moins soit à la lumière **visible,** soit à la lumière **ultraviolette.**
